# EUROPÄISCHE PATENTSCHRIFT

(11) **EP 0 385 210 B1**
(45) Veröffentlichungstag und Bekanntmachung des Hinweises auf die Patenterteilung: **15.02.1995**
(21) Anmeldenummer: 90103106.2
(22) Anmeldetag: 19.02.1990
(51) Int. Cl.: C07D 213/81

(54) **Amidierung von Pyridinen**
Amidation of pyridines
Amidification de pyridines

(30) Priorität: 28.02.1989 CH 736/89; 04.12.1989 CH 4323/89
(43) Veröffentlichungstag der Anmeldung: 05.09.1990
(60) Teilanmeldung: 93110620.7
(73) Patentinhaber: F. HOFFMANN-LA ROCHE AG, 4002 Basel (CH)
(72) Erfinder: Scalone, Michelangelo, Dr., CH-4127 Birsfelden (CH); Vogt, Peter, Dr., CH-4127 Birsfelden (CH)
(74) Vertreter: Kellenberger, Marcus, Dr.

(56) Entgegenhaltungen:
- DE-A- 2 305 758
- GB-A- 2 163 746
- US-A- 2 749 350
- CHEMICAL ABSTRACTS, Band 95, Nr. 9, 31. August 1981, Columbus, Ohio, USA AMESD.E. et al. " A conven- ient synthesis of ethynyl-N- heteroarenes" Seite 782,Spalte 2, Zusam- menfassung-Nr. 80 892s
- CHEMICAL ABSTRACTS, Band 99, Nr. 7, 15. August 1983, Columbus, Ohio, USASAKOMOTO T. et al. "A facile synthesis of ethynylsubsti- tuted six membered N-hetero- aromatic compounds" Seite 566, Spalte 1, Zusam- menfassung-Nr. 53 823w
- CHEMICAL ABSTRACTS, Band 73, 21. Dezember 1970, Columbus, Ohio, USA SHUARTSBERGM.S. et al. "New reaction of acetylene with terminal triple bonds" Seite 349,Spalte 1, Zusammenfassung-Nr. 130 851w

## Beschreibung

Die vorliegende Erfindung betrifft ein neues Verfahren zur Herstellung des Pyridin-2-carboxamids der allgemeinen Formel 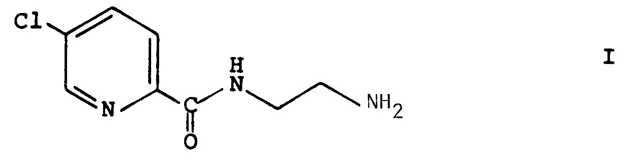
und von pharmazeutisch verwendbaren Saureadditionssalzen davon.

Die Verbindung der obigen Formel I ist das N-(2-Aminoäthyl)-5-chlorpyridin-2-carboxamid. Diese Verbindung sowie deren pharmazeutisch verwendbare Säureadditionssalze besitzen eine ausgeprägte, reversible und hoch selektive Monoaminooxidase B (MAO-B) hemmende Eigenschaft und eignen sich demnach zur Behandlung von depressiven Zuständen, Parkinsonismus und kognitiven Erkrankungen. Verfahren zur Herstellung von N-(2-Aminoäthyl)-5-chlorpyridin-2-carboxamid und dessen pharmazeutisch verwendbaren Salzen - unter anderem aus Verbindungen der obigen Formel I, worin R einen in Amino überführbaren Rest bedeutet - sowie deren interessante pharmakologische Eigenschaften sind beispielsweise in der Britischen Patentschrift Nr. 2163746 beschrieben.

Der in der vorliegenden Beschreibung verwendete Ausdruck "C₁₋₇-Alkyl" betrifft geradkettige und verzweigte Kohlenwasserstoffreste mit 1-7, vorzugsweise 1-4, Kohlenstoffatomen, wie Methyl, Aethyl, Propyl, Isopropyl, Butyl, Isobutyl, sec.-Butyl, t-Butyl, Pentyl, Hexyl, Heptyl und dergleichen. In gleicher Weise betreffen die Ausdrücke "C₁₋₈-Alkyl" und "C₁₋₄-Alkyl," entsprechende Kohlenwasserstoffreste mit 1-8 bzw. 1-4 Kohlenstoffatomen. Der Ausdruck "C₁₋₄-Alkoxy" bedeutet Alkyläthergruppe, worin "C₁₋₄-Alkyl" die obige Bedeutung hat. Der Ausdruck "Halogen" umfasst die vier Halogene Fluor, Chlor, Brom und Jod.

Das erfindungsgemässe Verfahren ist dadurch gekennzeichnet, dass man 2,5-Dichlorpyridin der Formel 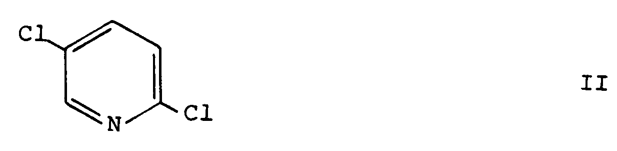
in Gegenwart eines Palladium-Phosphin-Katalysators
mit einem Alkin der allgemeinen Formel

R¹-C≡CH III

worin R¹ Wasserstoff, C₁₋₇-Alkyl, Trimethylsilyl oder die Gruppe -(R²)(R³)-COH und R² und R³ unabhängig voneinander je Wasserstoff oder C₁₋₇-Alkyl oder zusammen Cyclopentyl oder Cyclohexyl bedeuten,
umsetzt, das erhaltene Alkin der allgemeinen Formel 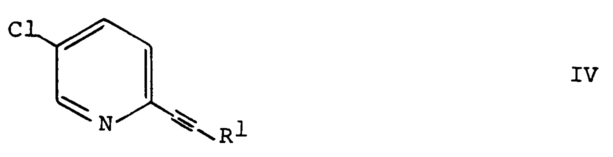
worin R¹ die oben angegebene Bedeutung besitzt, zur 5-Chlorpyridin-2-carbonsäure der Formel 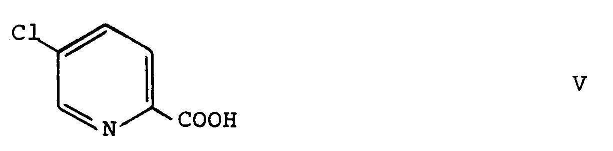
oxidiert und diese oder ein reaktionsfähiges funktionelles Derivat davon mit Äthylendiamin umsetzt und
erwünschtenfalls, die erhaltene Verbindung in ein pharmazeutisch verwendbares Säureadditionssalz überführt.

Die Alkinylierung von 2,5-Dichlorpyridin der Formel II mit einem Alkin der Formel III in Gegenwart eines Palladium-Phosphin-Katalysators kann man in an sich bekannter Weise durchführen. So kann man die Reaktion z.B. unter wasser- und sauerstofffreien Bedingungen in einer Inertgasatmosphäre in Gegenwart einer Base und einer katalytischen Menge von Kupfer-(I)-jodid in einem unter den Reaktionsbedingungen inerten Lösungsmittel oder Lösungsmittelgemisch bei einer Temperatur zwischen etwa 40° und 150°C, vorzugsweise zwischen etwa 60° und 100°C, durchführen. Geeignete Basen sind organische Basen, wie sekundäre oder tertiäre Amine. z.B. Dialkyl- oder Trialkylamine, wie Dimethylamin, Diäthylamin, Methyläthylamin, Trimethylamin, Triäthylamin, Aethyldiisopropylamin und dergleichen, und anorganische Basen, wie Natrium- oder Kaliumhydroxid, Calciumcarbonat und dergleichen, wobei überschüssiges Amin auch als Lösungsmittel dienen kann. Wird die Reaktion in Gegenwart einer anorganischen Base durchgeführt, kann die Verwendung eines Phasentransferkatalysators oder eines Kronenäthers von Vorteil sein. Die bevorzugte Base ist das Diäthylamin. Als Lösungsmittel kommen aliphatische und aromatische Kohlenwasserstoffe, wie Hexan, Benzol, Toluol, Xylol und dergleichen. halogenierte aliphatische und aromatische Kohlenwasserstoffe, wie Methylenchlorid, Chlorbenzol und dergleichen, Aether, wie Diäthyläther, Tetrahydrofuran, Dioxan und dergleichen, Ketone, wie Aceton, Methylpropylketon und dergleichen, Carbonsäureester, wie Methylacetat, Aethylacetat und dergleichen, Alkohole, wie t-Butanol und dergleichen, Dimethylformamid, Dimethylsulfoxid und dergleichen, in Frage. Bei der erfindungsgemässen Umsetzung ist der Druck nicht kritisch, weshalb man bei Atmosphärendruck oder erhöhten Drucken arbeiten kann, vorzugsweise bei Atmosphärendruck.

Beim Katalysator handelt es sich um eine Palladium-Phosphin-Komplexverbindung, welche gegebenenfalls auch in situ aus einer Palladiumkomponente und einem Phosphinliganden gebildet werden kann. Als Palladiumkomponente kommen in diesem Fall metallisches Palladium, das gegebenenfalls auf ein Trägermaterial, wie Kohle, aufgetragen ist, oder ein Komplex bzw. ein Salz des 0-, 2- oder 4-wertigen Palladiums, wie Palladium-dichlor-bis(actonitril), Palladium-bis(dibenzylidenaceton), Palladiumchlorid, Palladiumacetat und dergleichen, in Frage. Die Menge an Palladiumkomponente beträgt zweckmässigerweise 0,2-2,0 Mol %, vorzugsweise 0,2-0,5 Mol %. Als Phosphinligand kommen chirale und nicht chirale Mono- und Diphosphorverbindungen wie sie in Houben-Weyl, Methoden der organischen Chemie, Band El, Seite 106ff, Georg Thieme Verlag Stuttgart, 1982. und Aspects Homog. Catal., 4, 145-202 (1981) beschrieben sind, insbesondere solche der allgemeinen Formel

P(R⁴)(R⁵)(R⁶)

worin R⁴, R⁵ und R⁶ unabhängig voneinander je C₁₋₈-Alkyl, Cyclohexyl, Benzyl, Phenyl oder durch C₁₋₄-Alkyl, C₁₋₄-Alkoxy, Halogen, Trifluormethyl oder Phenyl substituiertes Phenyl bedeuten,
in Frage.

Die Menge an Phosphinligand beträgt zweckmässigerweise 0,1-100 Mol pro Mol Palladium, vorzugsweise 1-10 Mol pro Mol Palladium. Zur in situ Herstellung der Palladium-Phosphin-Komplexverbindung verwendet man vorzugsweise Palladium-dichlor-bis(acetonitril), Palladium-(II)-chlorid oder Palladium-(II)-acetat.

Bevorzugt ist jedoch die Verwendung einer bereits gebildeten Palladium-Phosphin-Komplexverbindung, wie Palladium-dichlor-bis(triphenylphosphin), Palladium-tetrakis(triphenylphosphin) und dergleichen, wobei Palladium-dichlor-bis(triphenylphosphin) die bevorzugte Komplexverbindung ist.

Die Oxidation eines erhaltenen Alkins der Formel IV kann man ebenfalls in an sich bekannter Weise mit Permanganat durchführen. So kann man die Reaktion in einem unter den Reaktionsbedingungen inerten Lösungsmittel oder Lösungsmittelgemisch bei einer Temperatur zwischen etwa 40° und 120°C, vorzugsweise zwischen etwa 60° und 100°C, durchführen. Geeignete Lösungsmittel sind Wasser und Gemische von Wasser mit mit Wasser mischbaren Aethern, wie Tetrahydrofuran, Dioxan und dergleichen, Ketonen, wie Aceton, Methyläthylketon und dergleichen, Acetonitril und dergleichen. Der Druck für diesen Reaktionsschritt ist nicht kritisch, weshalb man bei Atmosphärendruck oder erhöhten Drucken arbeiten kann, vorzugsweise jedoch bei Atmosphärendruck.

Die Umsetzung von 5-Chlorpyridin-2-carbonsäure mit Äthylendiamin ist beispielsweise in der oben erwähnten Britischen Patentschrift Nr. 2163746 vorbeschrieben.

Die Artikel in Synthesis 1981(5), 364-5 und 1983(4), 312-4 beschreiben u.a. die Uebeführung von Pyridin-monochlorid oder -monobromid in Alkinyl-pyridinderivaten. Im Gegensatz hierzu wird in der ersten Stufe des vorliegenden Verfahrens 2,5-Dichlorpyridin in 2-Alkinyl-5-chlorpyridinderivaten der Formel IV übergeführt.

Die US Patentschrift Nr. 2 749 350 beschreibt u.a. die Oxidation des alkylierten Alkinyl-pyridinderivates, 4-Äthinyl-3-methylpyridin, zur 3-Methylisonicotinsäure, nicht aber die in der zweiten Stufe des vorliegenden Verfahrens durchgeführte Oxidation des oben erwähnten chlorierten Alkinylpyridinderivates der Formel IV zur 5-Chlorpyridin-2-carbonsäure der Formel V.

Die Ueberführung der Verbindung der Formel I, in pharmazeutisch verwendbare Säureadditionssalze ist - wie bereits weiter oben erwähnt - beispielsweise in der Britischen Patentschrift Nr. 2163746 vorbeschrieben.

Das als Ausgangsmaterial verwendete 2,5-Dichlorpyridin sowie die als Ausgangsstoffe verwendeten Verbindungen der Formel III sind bekannt oder können in Analogie zu den bekannten Verbindungen hergestellt werden.

Die nachfolgenden Beispiele dienen der näheren Erläuterung der vorliegenden Erfindung; alle Temperaturen sind in Celsiusgraden angegeben.

### Beispiel 1

Ein 10 Liter-Sulfierkolben, ausgerüstet mit mechanischem Rühren, Thermometer, Intensivkühler, Tropftrichter, Einrichtung zur Gaseinleitung und Chlorcalciumrohr, wird unter Argon mit 1,850 kg (12,5 Mol) 2,5-Dichlorpyridin, 4,600 l Diäthylamin und 1,395 kg (16,25 Mol) 2-Methyl-3-butin-2-ol beschickt. Zur erhaltenen gelben Lösung gibt man unter Rühren 1,4 g (7,5 mMol) Kupfer-(I)-jodid sowie 26,3 g (37,5 mMol) Palladium-dichlor-bis(triphenylphosphin) zu. Die feine Suspension wird anschliessend zum Rückfluss (ca. 70°) erhitzt, und der Reaktionsverlauf dünnschicht- und gaschromatographisch verfolgt. Nach 24 Stunden wird das Reaktionsgemisch mit einem Eisbad auf 20° abgekühlt. Der Festkörper (Diäthylaminhydrochlorid) wird abfiltriert und dreimal mit je 700 ml Diäthylamin nachgewaschen. Der Festkörper enthält weder Produkt noch Palladium (<4 ppm) und wird verworfen. Die Filtrate werden vereinigt und unter vermindertem Druck bei 40° eingedampft. Dabei erhält man 2,905 kg eines dunkelbraunen, bei 20° fest werdenden Rückstands, dessen Reinheit gemäss GC (ISTD) 76,1% (g/g) beträgt.

950 g rohes Alkinol werden in 2,300 l Toluol gelöst, und diese Lösung wird in einem 5 Liter-Scheidetrichter vorgelegt. Dann werden zwei 3 Liter-Scheidetrichter mit je 500 ml Toluol beschickt. Anschliessend wird dreimal mit je 1 l entionisiertem Wasser unter jeweils guter Durchmischung durch die drei Scheidetrichter laufen gelassen. Die Wasserphasen werden verworfen, und die organischen Phasen werden in einem 6 Liter-Vierhalsrundkolben, ausgerüstet mit mechanischem Rühren und Thermometer, mit 12 g (0,25 Mol) Natriumcyanid, 600 ml entionisiertem Wasser und 1,2 g Tetrabutylammoniumbromid versetzt. Das erhaltene 2-phasige Gemisch wird 24 Stunden bei 25° kräftig gerührt. Dann werden die beiden Phasen getrennt, und die wässrige Phase mit 1 l Toluol gewaschen. Die beiden organischen Phasen werden nacheinander mit 2,5 l entionisiertem Wasser gewaschen. Die organischen Phasen werden vereinigt und über 250 g Natriumsulfat getrocknet, und die erhaltene Suspension abfiltriert. Der Festkörper wird mit 250 ml Toluol gewaschen, und die vereinigten Filtrate unter vermindertem Druck bei 40° eingedampft, wobei man 921 g 4-(5-Chlor-2-pyridyl)-2-methyl-3-butin-2-ol als dunkelbrauns Oel erhält, welches beim Stehenlassen kristallisiert. Dieses Rohprodukt (Rohausbeute; 115%; GC-Gehalt ca. 87%) wird direkt in die nächste Stufe eingesetzt.

450 g (2,0 Mol) Alkinol in einem 10 Liter-Sulfierkolben, ausgerüstet mit mechanischem Rührer, Thermometer, Rückflusskühler und Einrichtung zur Gaseinleitung, werden unter Argon mit 7 l warmem, entionisiertem Wasser (70-80°) versetzt, wobei unter Rühren eine gelbe Emulsion entsteht. Dann werden 1,043 kg (6,6 Mol) Kaliumpermanganat in 50 bis 75 g-Portionen so zugegeben, dass die Innentemperatur 70° bis 80° beträgt, Dauer der Zugabe: ca. 2 Stunden. Der Reaktionsverlauf wird dünnschichtchromatographisch verfolgt. Bis zum Nachweis eines vollständigen Umsatzes wird das Reaktionsgemisch mit einem heissen Oelbad (100°) bei ca. 80° gehalten. Dann wird das Reaktionsgemisch heiss abfiltriert, und das Mangandioxid sechsmal mit je 1 l heissem entionisiertem Wasser (ca. 90°) gewaschen. Das Filtrat wird unter vermindertem Druck bei 40° auf ein Volumen von 7 l eingeengt. Spuren von Braunstein im Konzentrat werden durch Filtrieren über feines Papier entfernt.

Zum Filtrat in einem 10 Liter-Sulfierkolben, ausgerüstet mit mechanischem Rührer, Thermometer, Rückflusskühler, pH-Elektrode, Tropftrichter und Einrichtung zur Gaseinleitung, werden unter Argon und Rühren bei 20° 365 ml Salzsäure (rein) langsam getropft bis zum Erreichen von pH 3. Nach beendeter Zugabe der Salzsäure (ca. 45 Minuten) wird die Suspension im Eisbad auf 4° abgekühlt und eine Stunde nachgerührt. Der Festkörper wird anschliessend abfiltriert und mit 2,100 l entionisiertem Wasser (eiskalt) gewaschen. Der Rückstand wird über Nacht unter vermindertem Druck bei 50° getrocknet. Man erhält 289 g (90%) 5-Chlorpyridin-2-carbonsäure als hellbeiges Pulver.

567 g (3,6 Mol) 5-Chlorpyridin-2-carbonsäure in einem 10 Liter-Vierhalssulfierkolben, ausgerüstet mit mechanischem Rührer, Thermometer, Rückflusskühler, Tropftrichter mit Druckausgleich und Einrichtung zur Gaseinleitung, werden unter Argon mit 4,540 l 2-Butanol beschickt. Zur erhaltenen Lösung gibt man unter Rühren 67 ml konz. Schwefelsäure und heizt das Gemisch anschliessend zum Rückfluss, wobei der Reaktionsverlauf gaschromatographisch verfolgt wird. Nach 3,5 Stunden sind gemäss GC noch ca. 4% Säure im Gemisch enthalten. Der aufsteigende Kühler wird durch einen absteigenden ersetzt, und man tropft 1 l 2-Butanol innerhalb von 2,5 Stunden zu, während gleichzeitig 2 l Lösungsmittel aus dem Reaktionsgemisch abdestilliert werden. Mit GC werden 97% Ester und 1,5% Säure im Reaktionsgemisch nachgewiesen. Nach Abdestillieren von weiteren 1,5 l Lösungsmittel wird das Reaktionsgemisch mit einem Eisbad aud 25° abgekühlt und in einem 20 Liter-Ausrührgefäss mit 4 l Toluol und einer Lösung aus 303 g Natriumbicarbonat in 4 l entionisiertem Wasser versetzt. Weitere drei 20 Liter-Ausrührgefässe werden mit je 1 l Toluol beschickt, und die organischen Phasen mit der Wasserphase aus dem ersten Ausrührgefäss sowie dreimal je 1 l entionisiertem Wasser der Reihe nach gewaschen. Die Wasserphasen werden verworfen. Die organischen Phasen werden vereinigt und über 1 kg Natriumsulfat getrocknet. Das Trocknungsmittel wird abfiltriert, und das Filtrat unter vermindertem Druck bei 30°-50° eingedampft. Man erhält 809 g braunes Oel als Rohprodukt. 767 g Rohprodukt werden einer Destillation am Hochvakuum unterworfen. Die Hauptfraktion siedet bei 86-89°/0,1 Pa. Man erhält auf diese Weise 693 g hellgelbes Destillat (94%) von 5-Chlorpyridin-2-carbonsäure-2-butylester, welches bei 25° eine ölige, feste Masse bildet, GC-Reinheit: 98,3%.

Ein 10 Liter-Vierhalssulfierkolben, ausgerüstet mit mechanischem Rührer, Thermometer und Einrichtung zur Gaseinleitung, wird unter Argon mit 692 g (3,2 Mol) 5-Chlorpyridin-2-carbonsäure-2-butylester und 7 l Aethylendiamin beschickt, und die erhaltene, klare Lösung verrührt, wobei die Innentemperatur während des Nachrührens langsam von 22° auf maximal 30° ansteigt. Der Reaktionsverlauf wird gaschromatographisch verfolgt. Nach 3 Stunden Reaktionszeit (die Innentemperatur beträgt noch 27°) wird die Reaktionslösung unter vermindertem Druck bei 30°-40° eingedampft. Der Eindampfrückstand - ein klares, gelbes Oel - wiegt 753 g. Ein 20 Liter Ausrührgefäss sowie zwei 5 Liter-Scheidetrichter werden mit 2,250 l eiskalter 3N Salzsäure bzw. mit 750 ml 1,5N Salzsäure bzw. mit 750 ml entionisiertem Wasser beschickt. Der Eindampfrückstand (753 g eines klaren, gelbes Oels) wird anschliessend mit 1,500 l Methylenchlorid versetzt, und die erhaltene Lösung in das Ausrührgefäss gegeben. Nach dem Durchmischen der beiden Phasen beträgt der pH-Wert der Wasserphase ca. 1. Anschliessend werden die organische Phase aus dem Ausrührgefäss sowie zweimal je 1,5 l Methylenchlorid der Reihe nach und unter guter Durchmischung durch das Ausrührgefäss und die beiden Scheidetrichter geschickt. Die Wasserphasen werden im Ausrührgefäss vereinigt und durch Zugabe von 3 l eiskalter 3N Natronlauge auf ca. pH 11 gestellt. Die beiden Scheidetrichter werden mit zweimal je 750 ml halbgesättigter Kochsalzlösung beschickt. Dann werden unter jeweils guter Durchmischung sechsmal je 1,5 l Methylenchlorid durch das Ausrührgefäss und die beiden Scheidetrichter geschickt. Die organischen Phasen werden vereinigt und über 500 g Natriumsulfat getrocknet. Das Trocknungsmittel wird abfiltriert, und das Filtrat unter vermindertem Druck bei 30°-40° eingedampft. Der gelbe, ölige Rückstand wird noch 15 Stunden bei 40°/0,1 Pa getrocknet und wiegt danach 616 g (3,08 Mol). Er kristallisiert durch beim Stehenlassen bei 20°. Das Kristallisat wird bei 20° in 3,2 l Methanol gelöst. Zur Lösung werden unter Kühlen im Eisbad 688 ml 4,48N methanolische Salzsäure (3,08 Mol) auf einmal gegeben. Das Reaktionsgemisch wird 15 Minuten nachgerührt, und die entstandene Suspension anschliessend auf 50° erwärmt, wobei erneut eine klare Lösung entsteht. Dazu werden innerhalb von 30 Minuten 5,8 l t-Butylmethyläther (vorgewärmt auf 50°) gegeben. Die erhaltene Suspension lässt man auf 20° abkühlen (Dauer ca. 1,5 Stunden), und anschliessend wird sie mit einem Eisbad auf 0°-5° abgekühlt. Der Festkörper wird anschliessend abfiltriert und mit 1,100 l Petroläther (tiefsiedend) nachgewaschen. Das Kristallisat wird anschliessend im Vakuumtrockenschrank bei 40° bis zur Gewichtskonstanz getrocknet und wiegt danach 693 g (95% Kristallisationsausbeute). Das Kristallisat wird zur weiteren Reinigung in 3,6 l Methanol auf 55° erhitzt und gelöst. Unter kräftigem Rühren lässt man innerhalb von 1,5 Stunden 5,500 l t-Butyl-methyläther (vorgewärmt auf 50°) zulaufen, wobei nach Zugabe von 1,5 l Lösungsmittel mit reinen Kristallen des Endproduktes angeimpft wird. Die erhaltene Suspension lässt man auf ca. 20° abkühlen (Dauer ca. 1,5 Stunden), worauf mit einem Eisbad aud 0°-5° gekühlt wird. Das Kristallisat wird abfiltriert und mit 1,1 l Pentan gewaschen. Das erhaltene N-(2-Aminoäthyl)-5-chlorpyridin-2-carboxamid-hydrochlorid wird im Hochvakuum bei 50°/1 Pa während zweier Tage getrocknet und wiegt danach 665 g (87%), Smp. 197-199°.

### Beispiel 2

Unter Argon werden 9,0 ml (80 mMol) 1-Hexin und 6,0 g (40 mMol) 2,5-Dichlorpyridin in 80 ml Diäthylamin vorgelegt, wobei eine hellgelbe Lösung entsteht. Dazu gibt man 0,40 g (1,5 mMol) Triphenylphosphin, 0,04 g (0,2 mMol) Kupfer-(I)-jodid sowie 0,24 g (0,9 mMol) Palladium-dichlor-bis(triphenylphosphin). Die erhaltene orange Lösung wird anschliessend unter Rühren 24 Stunden zum Rückfluss erhitzt. Danach wird das Reaktionsgemisch auf 25° abgekühlt und unter vermindertem Druck bei 40° eingedampft. Der braune Rückstand wird dann mit 50 ml Aethanol versetzt und erneut eingedampft. Das Rohprodukt wird über 50 g Kieselgel mit 700 ml Hexan/Toluol (1:1) filtriert, und das Eluat unter vermindertem Druck bei 50° eingedampft. Der Rückstand wird anschliessend im Kugelrohr bei 190°/2600 Pa destilliert. Man erhält 6,8 g 2-Hexinyl-5-chlorpyridin als gelbes Oel.

### Beispiel 3

Unter Argon werden 1,2 ml (10 mMol) Trimethylsilylacetylen, 40 ml Diäthylamin und 1,5 g (10 mMol) 2,5-Dichlorpyridin bei 20° gerührt. Zur erhaltenen farblosen Lösung gibt man 0,1 g (0.4 mMol) Triphenylphosphin, 0,01 g (0,05 mMol) Kupfer-(I)-jodid sowie 0,06 g (0,25 mMol) Palladium-dichlor-bis(acetonitril). Die Reaktionslösung wird anschliessend unter Rühren 5 Stunden zum Rückfluss erhitzt. Nach Abkühlen auf 25° wird die Reaktionslösung unter vermindertem Druck bei 40° eingedampft. Zum Rückstand gibt man anschliessend 50 ml Aethanol und dampft erneut ein. Das Rohprodukt wird über 30 g Kieselgel mit Essigester filtriert, und das Eluat eingedampft, wobei 1,6 g braune Flüssigkeit verbleiben. Davon wird 1,0 g im Kugelrohr bei 70°/0,1 Pa destilliert, wobei als Hauptfraktion 0,6 g 2-Trimethylsilyläthinyl-5-chlorpyridin erhalten werden, welches beim Stehenlassen erstarrt.

Unter Inertgasatmosphäre werden 0,10 g (0,5 mMol) 2-Trimethylsilyläthinyl-5-chlorpyridin, 0,01 g Natriumlaurylsulfat und 2 ml entionisiertes Wasser vorgelegt. Anschliessend gibt man unter Rühren bei 23° 0,25 g (15 mMol) Kaliumpermanganat zu. Nach 2 Stunden wird das Reaktionsgemisch mit wässriger Natriumbisulfitlösung (ca. 40-proz.) versetzt, bis nach Filtrieren einer Probe über Filterpapier die violette Farbe des Permanganates verschwunden ist. Die Suspension wird über eine Schicht Dicalite filtriert, und das Filtrat mit 1N wässriger Salzsäure auf pH 3 gestellt. wobei eine weisse Suspension entsteht. Diese wird im Eisbad gekühlt, und die Kristalle werden abfiltriert. Die Kristalle werden mit 1 ml entionisiertem Wasser versetzt, die Suspension erneut auf 0° abgekühlt, und die Kristalle abfiltriert. Diese werden unter vermindertem Druck zweimal aus Methanol/Toluol eingedampft, und der Rückstand am Hochvakuum getrocknet. Man erhält 17 mg 5-Chlorpyridin-2-carbonsäure als weissen, festen Rückstand, Smp. 171-172°.

## Patentansprüche

1. Verfahren zur Herstellung des Pyridin-2-carboxamids der allgemeinen Formel 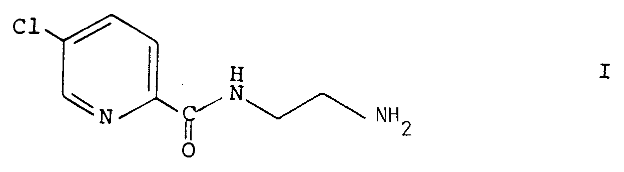 und von pharmazeutisch verwendbaren Säureadditionssalzen davon, dadurch gekennzeichnet, dass man 2,5-Dichlorpyridin der Formel 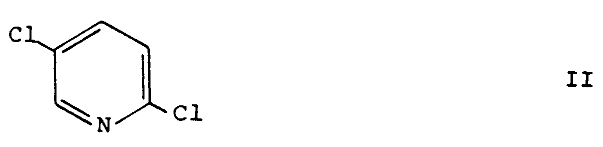 in Gegenwart eines Palladium-Phosphin-Katalysators
mit einem Alkin der allgemeinen Formel
R¹-C≡CH III
worin R¹ Wasserstoff, C₁₋₇-Alkyl, Trimethylsilyl oder die Gruppe -(R²)(R³)-COH und R² und R³ unabhängig voneinander je Wasserstoff oder C₁₋₇-Alkyl oder zusammen Cyclopentyl oder Cyclohexyl bedeuten,
umsetzt, das erhaltene Alkin der allgemeinen Formel 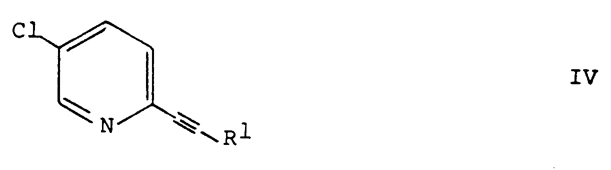 worin R¹ die oben angegebene Bedeutung besitzt, zur 5-Chlorpyridin-2-carbonsäure der Formel 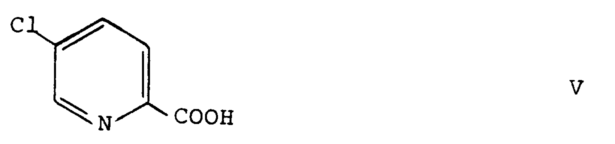 oxidiert und diese oder ein reaktionsfähiges funktionelles Derivat davon mit Äthylendiamin umsetzt und
erwünschtenfalls, die erhaltene Verbindung in ein pharmazeutisch verwendbares Säureadditionssalz überführt.

2. Verfahren nach Anspruch 1, dadurch gekennzeichnet, dass man die Reaktion in Gegenwart einer Base durchführt.

3. Verfahren nach Anspruch 1 oder 2, dadurch gekennzeichnet, dass bei der Alkinylierung das Diäthylamin auch als Lösungsmittel dient.

4. Verfahren nach einem der Ansprüche 1-3, dadurch gekennzeichnet, dass man die Alkinylierung bei einer Temperatur zwischen etwa 40° und 150°C durchführt.

5. Verfahren nach Anspruch 4, dadurch gekennzeichnet, dass man die Alkinylierung bei einer Temperatur zwischen etwa 60° und 100°C durchführt.

6. Verfahren nach einem der Ansprüche 1-5, dadurch gekennzeichnet, dass man die Umsetzungen bei einem Druck von etwa 1o⁵ bis etwa 10⁷ Pa durchführt.

7. Verfahren nach Anspruch 6, dadurch gekennzeichnet, dass man die Alkinylierung bei einem Druck von etwa 10⁵ Pa durchführt.

8. Verfahren nach einem der Ansprüche 1-7, dadurch gekennzeichnet, dass man die Umsetzung in Gegenwart von Palladium-dichlor-bis(triphenylphosphin) durchführt.

9. Verfahren nach einem der Ansprüche 1-8, dadurch gekennzeichnet, dass die Oxidation mit Kaliumpermanganat erfolgt.

10. Verfahren nach Anspruch 9, dadurch gekennzeichnet, dass die Oxidation bei einer Temperatur zwischen etwa 70° und 80°C erfolgt.

11. Verfahren nach Anspruch 9 oder 10, dadurch gekennzeichnet, dass die Oxidation in Wasser durchgeführt wird.

## Claims

1. A process for the manufacture of the pyridine-2-carboxamide of the general formula 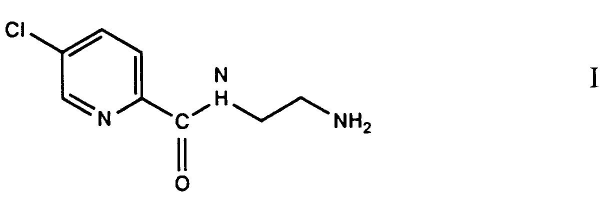 and of pharmaceutically usable acid addition salts thereof, characterized by reacting 2,5-dichloropyridine of the formula 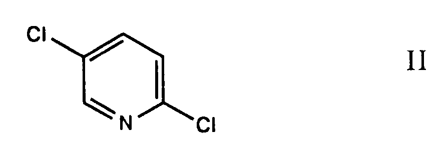 in the presence of a palladium-phosphine catalyst with an alkyne of the general formula
R¹-C≡CH III
wherein R¹ signifies hydrogen, C₁₋₇-alkyl, trimethylsilyl or the group -(R²)(R³)-COH and R² and R³ each independently signify hydrogen or C₁₋₇-alkyl or together signify cyclopentyl or cyclohexyl,
oxidizing the resulting alkyne of the general formula 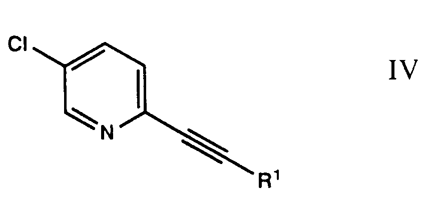 wherein R¹ has the significance given above, to give 5-chloropyridine-2-carboxylic acid of the formula 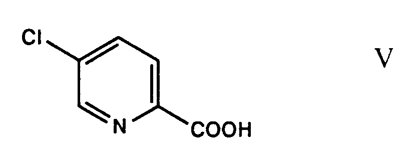 and reacting this or a reactive functional derivative thereof with ethylenediamine and if desired, converting the obtained compound into a pharmaceutically usable acid addition salt.

2. A process according to claim 1, characterized in that the reaction is carried out in the presence of a base.

3. A process according to claim 1 or 2, characterized in that diethylamine also serves as the solvent in the alkynylation.

4. A process according to any one of claims 1-3, characterized in that the alkynylation is carried out at a temperature between about 40° and 150°C.

5. A process according to claim 4, characterized in that the alkynylation is carried out at a temperature between about 60° and 100°C.

6. A process according to any one of claims 1-5, characterized in that the reactions are carried out at a pressure of about 10⁵ to about 10⁷ Pa.

7. A process according to claim 6, characterized in that the alkynylation is carried out at a pressure of about 10⁵ Pa.

8. A process according to any one of claims 1-7, characterized in that the reaction is carried out in the presence of palladium-dichloro-bis(triphenylphosphine).

9. A process according to any one of claims 1-8, characterized in that the oxidation is effected with potassium permanganate.

10. A process according to claim 9, characterized in that the oxidation is effected at a temperature between about 70° and 80°C.

11. A process according to claim 9 or 10, characterized in that the oxidation is carried out in water.

## Revendications

1. Procédé de préparation du pyridine-2-carboxamide de formule générale 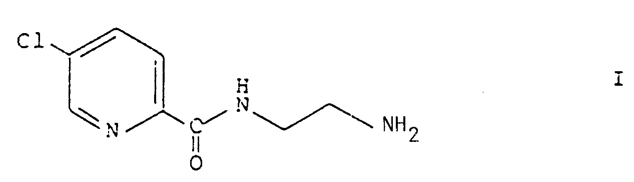 et de ses sels d'addition d'acides pharmaceutiquement acceptables, caractérisé en ce qu'on fait réagir la 2,5-dichloropyridine de formule 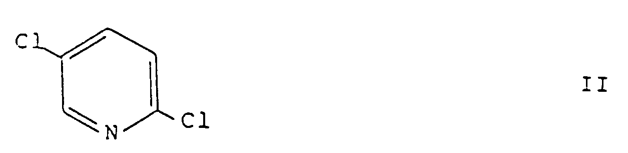 en présence d'un catalyseur de palladium-phosphine avec un alcyne de formule générale
R¹-C≡CH III
dans laquelle R¹ représente un hydrogène, un alkyle en C₁ à C₇, un triméthylsilyle ou le groupe -(R²)(R³ )-COH et R² et R³ représentent chacun indépendamment l'un de l'autre un hydrogène ou un alkyle en C₁ à C₇ ou représentent ensemble un cyclopentyle ou un cyclohexyle, en ce qu'on oxyde l'alcyne obtenu de formule générale 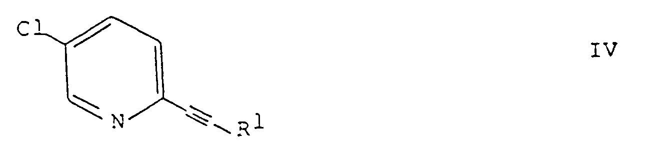 dans laquelle R¹ a la signification donnée ci-dessus, en acide 5-chloropyridine-2-carboxylique de formule 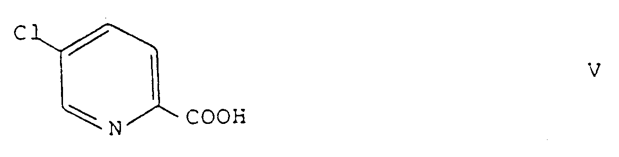 et en ce qu'on fait réagir celui-ci, ou un de ses dérivés fonctionnels réactifs, avec l'éthylènediamine et, si on le désire, en ce qu'on tranforme le composé obtenu en un sel d'addition d'acide pharmaceutiquement acceptable.

2. Procédé selon la revendication 1, caractérisé en ce qu'on conduit la réaction en présence d'une base.

3. Procédé selon la revendication 1 ou 2, caractérisé en ce que dans l'alcynylation la diéthylamine sert également de solvant.

4. Procédé selon l'une des revendications 1-3, caractérisé en ce qu'on conduit l'alcynylation à une température comprise entre environ 40° et 150°C.

5. Procédé selon la revendication 4, caractérisé en ce qu'on conduit l'alcynylation à une température comprise entre environ 60° et 100°C.

6. Procédé selon l'une des revendications 1-5, caractérisé en ce qu'on conduit les réactions à une pression d'environ 10⁵ à environ 10^{7.} Pa.

7. Procédé selon la revendication 6, caractérisé en ce qu'on conduit l'alcynylation à une pression d'environ 10⁵ Pa.

8. Procédé selon l'une des revendications 1-7, caractérisé en ce qu'on conduit la réaction en présence de palladium-dichloro-bis(triphénylphosphine).

9. Procédé selon l'une des revendications 1-8, caractérisé en ce que l'oxydation s'effectue avec du permanganate de potassium.

10. Procédé selon la revendication 9, caractérisé en ce que l'oxydation s'effectue à une température comprise entre environ 70° et 80°C.

11. Procédé selon la revendication 9 ou 10, caractérisé en ce qu'on conduit l'oxydation dans l'eau.
